Europäisches Patentamt

European Patent Office

Office européen des brevets

(19)

(11) Veröffentlichungsnummer: **0 177 661 B1**

(12) # EUROPÄISCHE PATENTSCHRIFT

(45) Veröffentlichungstag der Patentschrift:
**09.03.88**

(51) Int. Cl.⁴: **A 61 N 1/36**

(21) Anmeldenummer: **84890186.4**

(22) Anmeldetag: **10.10.84**

(54) Gerät zur Vestibularisreizung.

(43) Veröffentlichungstag der Anmeldung:
**16.04.86 Patentblatt 86/16**

(45) Bekanntmachung des Hinweises auf die Patenterteilung:
**09.03.88 Patentblatt 88/10**

(84) Benannte Vertragsstaaten:
**BE CH DE FR GB IT LI LU NL SE** ·

(56) Entgegenhaltungen:
**FR - A - 2 513 514**
**US - A - 3 540 453**
**US - A - 3 897 789**

(73) Patentinhaber: **Mark, Hermann, Dr., Favoritenstrasse 27, A-1040 Wien (AT)**

(72) Erfinder: **Mark, Hermann, Dr., Favoritenstrasse 27, A-1040 Wien (AT)**

(74) Vertreter: **Piso, Eberhard, Dr., Patentanwälte Dipl.-Ing. Herbert C.E. Krause Dr. Eberhard Piso Gluckgasse 1 Fach 328, A-1010 Wien 1 (AT)**

## Beschreibung

Die Erfindung betrifft ein Gerät zur Vestibularisreizung. Die DE-AS 1 229 658 zeigt eine Vorrichtung mit einem Niederfrequenzoszillator zur Heilbehandlung von Erkrankungen des Gehörsystems. Bei dieser Vorrichtung werden Impulse im Inneren der Gehörschnecke abgegeben.

Aus der DE-OS 2 948 267 ist eine Stimulationsvorrichtung bekannt, die mittels elektrischer Signale eine Verbesserung des Sehvermögens von Sehbehinderten herbeiführen soll.

In der DE-OS 2 635 826 wird eine Vorrichtung beschrieben, die pulsierende Hochfrequenzsignale von 450 kHz und 1,65 MHz erzeugt. Es soll durch eine selektive Stimulierung mehrerer Elektroden, die mit einem Empfänger gekoppelt sind, eine Schmerzunterdrückung in den geplagten Regionen durchgeführt werden, wobei die Elektroden auf bestimmte Nerven hin ausgerichtet sind.

Keine dieser Vorrichtungen eignet sich aber für eine Vestibularisreizung insbesondere zur Erzeugung eines Gleichgewichtsreizes.

Die Aufgabe der Erfindung ist es, ein kleines, leicht tragbares und handliches Gerät zur Vestibularisreizung zu schaffen. Es bringt seinem Träger bei allen Arten, der unter dem Begriff Kinetosen zusammengefassten Krankheiten, so z.B. bei Raumkrankheit, Seekrankheit, Autokrankheit, Luftkrankheit, usw., sowie bei Schwindelbeschwerden, durch Erzeugung eines Gleichgewichtsreizes Linderung seines Leidens.

Die Erfindung löst die Aufgabe dadurch, dass mit einer Stromquelle ein Taktgenerator zur Erzeugung von Impulsen mit gegebenenfalls variabler Frequenz, ein Impulsgenerator zum Einstellen der Impulsdauer, ein Impulsformer zum Einstellen der Flankensteilheit und eine Treiberstufe zum Einstellen der Amplitude in Reihe geschaltet sind und die Treiberstufe mit im Schädelbereich eines Patienten anbringbaren Elektroden verbunden ist.

Eine weitere Ausgestaltung der Erfindung ist dadurch gegeben, dass die Stromquelle eine Gleichstrombatterie, ein Akkumulator oder ein Netzanschluss ist.

Weiters besteht eine weitere Ausgestaltung der Erfindung auch darin, dass die Impulsform trapezförmig ist, dass die Impulsform kurvenförmig ist, dass die Impulsform dreieckförmig ist, dass die Impulsform rechteckförmig ist, dass die Impulse auf- und abschwellend sind oder dass die Impulse von dauernder gleichmässiger Folge sind oder dass die Impulse stossweise von den Elektroden abgegeben werden.

Ausserdem besteht eine weitere Ausgestaltung der Erfindung auch darin, dass der Taktgenerator zwischen 1 und 10 Impulsen pro Sekunde varriierbar ist, dass der Impulsgenerator zwischen 100 und 300 msek einstellbar ist und dass die Stromstärke in der Treiberstufe zwischen 0,5 und 10, vorzugsweise zwischen 0,5 und 3,8 mA, einstellbar ist.

Überdies besteht eine weitere Ausgestaltung der Erfindung darin, dass die Variablen als Potentiometer ausgebildete Widerstände sind, dass die Gerätekomponenten miteinander in Wirkverbindung stehende Module sind, dass die Module Einheiten beinhalten, die programmierbar sind und dass die einzelnen Module durch gedruckte oder verdrahtete Schaltungen miteinander in Wirkverbindung stehen.

Weiters besteht eine weitere Ausgestaltung der Erfindung auch darin, dass die Elektroden bügelartig ausgebildet sind, oder dass die Elektroden durch ein Stirnband oder ein Federband an dem Schädel, vorzugsweise hinter den Ohren, angelegt sind, oder dass der Elektrodenträger ein Helm ist oder dass die Elektroden, vorzugsweise im Schädelbereich eingepflanzt oder angeklebt sind oder auch dadurch, dass die Elektroden an der Geräteaussenseite befestigt sind und mit diesem direkt an dem Körper des Patienten befestigt sind. Vor allem bei Raumkrankheit im schwerelosen Zustand sollen durch dieses erfindungsgemässe Gerät scheinbare Bedingungen wie beim Normalzustand in der Gravitationszone der Erde geschaffen werden. Das stimulierte Schweregefühl schützt den Astronauten vor vermeidbaren physischen und psychischen Übelständen, wie sie im schwerelosen Zustand durch den Verlust des Körpergewichts zustande kommen. Das künstliche Schweregefühl verhindert bei den Astronauten das Auftreten der Raumkrankheit und schützt sie vor dadurch herforgerufenen Bedienungs- und Kontrollfehlern mit möglichen, meist nicht wieder gutzumachenden Folgen.

Weiters wird durch dieses erfindungsgemässe Vestibularisreizgerät der Personenkreis, der bei Weltraumexperimenten der Schwerelosigkeit ausgesetzt wird, erheblich erweitert, da bisher viele der in Frage kommenden Menschen zumindest unter Übelkeit im schwerelosen Zustand zu leiden hatten und allein schon aus diesem Grund aus der engeren Wahl ausscheiden mussten. Auch bei Seekrankheit und Schwindelgefühl kann das erfindungsgemässe Gerät eingesetzt werden und dadurch einem noch wesentlich grösseren Kreis von Menschen dienlich sein.

Ausserdem erübrigt sich dadurch eine medikamentöse Behandlung mit möglichen Nebenwirkungen. Die Elektroden des erfindungsgemässen Vestibularisreizgerätes können im Schädelbereich durch z.B. ein Stirnband, einen Hut oder wie bei Astronauten durch einen Helm angelegt werden. Sie können aber auch in einem Bügel integriert sein oder unterhalb der Haut im Körper eingepflanzt oder auch auf der Haut, beispielsweise durch Ankleben oder mit Saugnäpfen, befestigt werden.

Die zu verwendende Stromstärke richtet sich vorwiegend nach dem Hautwiderstand und der Schwere des Leidens, liegt aber im ungefähren Bereich von 0,5 bis 3,8 mA. Vorzugsweise wird ein trapezförmiger Impuls, der z.B. dauernd gleichförmig dem Patienten über elektrische Leitungen zugeführt wird, verwendet, wobei es jedoch auch möglich ist, mittels eines Senders und eines Empfängers am bzw. im Körper des Patienten eine drahtlose Übertragung des entsprechend geformten Impulses durchzuführen.

Dieser Impuls muss in allen seinen Komponenten, z.B. der Flankensteilheit, der Dauer, der Impulsfolge, oder in seiner Stärke usw. dem Patienten angepasst werden. Dies kann z.B. mit einem Testgerät geschehen, das die nötigen Werte auswirft und möglicherweise sogar an einen Kleincomputer weitergibt, der Chips, die in den möglichen Modulen des erfindungsgemässen Gerätes enthalten sind, mit den geeigneten Werten programmiert und dadurch die bestmögliche Variante für den jeweiligen Patienten und den jeweiligen Zeitpunkt schafft. Die besten Ergebnisse erzielt man im allgemeinen mit einer Impulsdauer von100 bis 300 msek und einer Impulszahl von 1 bis 5 Impulsen pro sek.

Weiters ist es möglich, dass die Impulse gleichzeitig oder zeitlich verschoben im Bereich beider Ohren oder nur eines Ohres des Patienten appliziert werden.

Das erfindungsgemässe Vestibularisreizgerät kann aber auch als einheitliche Schaltung ohne Module ausgeführt werden, wobei dann die Werte beispielsweise vom behandelnden Arzt an den variablen Schaltungsteilen als Grundeinstellung eingegeben werden können. Dabei soll auch dem Patienten eine Selbstregelung eingeräumt werden, die er dann je nach Bedarf durchführen kann.

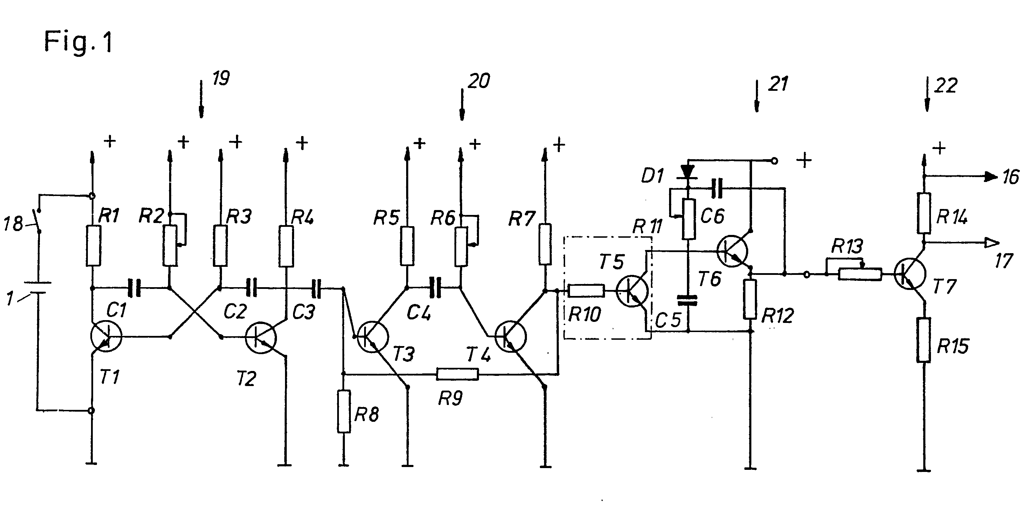

Die Erfindung ist in der Zeichnung anhand eines Ausführungsbeispiels näher veranschaulicht. Die Figur zeigt einen möglichen Schaltplan des erfindungsgemässen Vestibularisreizgerätes. Aus der Zeichnung ist zu ersehen, dass das erfindungsgemässe Gerät von einer Stromquelle 1 gespeist wird, an die der Taktgenerator 19 über einen Hauptschalter 18 angeschlossen ist. Die Transistoren T1, T2, die Widerstände R1 bis R4 und die Kondensatoren C1 und C2 bilden den Taktgenerator 19, dessen Frequenz durch den als Potentiometer ausgeführten Widerstand R2 verändert werden kann.

Die vom Taktgenerator 19 erzeugte Spannung wird über den Kondensator C3 ausgekuppelt und triggert den aus den Transistoren T3, T4, den Widerständen R5 bis R9 und den Kondensator C4 gebildeten monostabilen Multivibrator 20, der den Impulsgenerator darstellt. Der Widerstand R6 ist als Potentiometer ausgeführt und bestimmt je nach Wunsch die Impulsdauer des Impulsgenerators 20. Die von vorgenannter Stufe erzeugten Impulse steuern über den Widerstand R10 den Schalttransistor T5, der seinerseits die aus dem Transistor T6, den Widerständen R11 und R12, den Kondensatoren C5 und C6 sowie der Diode D1 gebildeten Impulsformerstufe 21 steuert. Die Impulsformerstufe 21 leitet aus den im Impulsgenerator 20 erzeugten Impulsen eine Trapezspannung ab, deren Flankensteilheit mit dem als Potentiometer ausgeführten Widerstand R11 verändert werden kann. Der veränderbare Widerstand R13 bestimmt den Basisstrom des Transistors T7 und somit die gewünschte Elektrodenspannung an den Elektroden 16 und 17, welche in der aus dem Transistor T7, den Widerständen R14 und R15 gebildeten Treiberstufe 22 erzeugt wird.

## Patentansprüche

1. Gerät zur Vestibularisreizung, dadurch gekennzeichnet, dass mit einer Stromquelle (1) ein Taktgenerator (19) zur Erzeugung von Impulsen mit gegebenenfalls variabler Frequenz, ein Impulsgenerator (20) zum Einstellen der Impulsdauer, ein Impulsformer (21) zum Einstellen der Flankensteilheit und eine Treiberstufe (22) zum Einstellen der Amplitude in Reihe geschaltet sind und die Treiberstufe (22) mit im Schädelbereich eines Patienten anbringbaren Elektroden (16, 17) verbunden ist.

2. Gerät zur Vestibularisreizung nach Anspruch 1, dadurch gekennzeichnet, dass die Stromquelle (1) eine Gleichstrombatterie oder ein Akkumulator ist.

3. Gerät zur Vestibularisreizung nach Anspruch 1, dadurch gekennzeichnet, dass die Stromquelle (1) ein Netzanschluss ist.

4. Gerät zur Vestibularisreizung nach einem der Ansprüche 1 bis 3, dadurch gekennzeichnet, dass die Impulsform trapezförmig ist.

5. Gerät zur Vestibularisreizung nach einem der Ansprüche 1 bis 3, dadurch gekennzeichnet, dass die Impulsform kurvenförmig ist.

6. Gerät zur Vestibularisreizung nach einem der Ansprüche 1 bis 3, dadurch gekennzeichnet, dass die Impulsform dreieckförmig ist.

7. Gerät zur Vestibularisreizung nach einem der Ansprüche 1 bis 3, dadurch gekennzeichnet, dass die Impulsform rechteckförmig ist.

8. Gerät zur Vestibularisreizung nach einem der Ansprüche 1 bis 7, dadurch gekennzeichnet, dass die Impulse auf- und abschwellend sind.

9. Gerät zur Vestibularisreizung nach einem der Ansprüche 1 bis 7, dadurch gekennzeichnet, dass die Impulse von dauernder gleichmässiger Folge sind.

10. Gerät zur Vestibularisreizung nach einem der Ansprüche 1 bis 7, dadurch gekennzeichnet, dass die Elektroden (16, 17) Impulse stossweise abgeben.

11. Gerät zur Vestibularisreizung nach einem der Ansprüche 1 bis 10, dadurch gekennzeichnet, dass der Taktgenerator (19) zwischen 1 und 10 Impulsen pro Sekunde varrierbar ist.

12. Gerät zur Vestibularisreizung nach einem der Ansprüche 1 bis 11, dadurch gekennzeichnet, dass der Impulsgenerator (20) zwischen 100 und 300 msek einstellbar ist.

13. Gerät zur Vestibularisreizung nach einem der Ansprüche 1 bis 12, dadurch gekennzeichnet, dass die Stromstärke in der Treiberstufe (22) zwischen 0,5 und 10 mA, vorzugsweise zwischen 0,5 und 3,8 mA, einstellbar ist.

14. Gerät zur Vestibularisreizung nach einem der Ansprüche 1 bis 13, dadurch gekennzeichnet, dass die Variablen als Potentiometer ausgebildete Widerstände (R2, R6, R11, R13) sind.

15. Gerät zur Vestibularisreizung nach einem der Ansprüche 1 bis 14, dadurch gekennzeichnet, dass die Gerätekomponenten (19, 20, 21, 22) miteinander in Wirkverbindung stehende Module sind.

16. Gerät zur Vestibularisreizung nach einem der Ansprüche 1 bis 15, dadurch gekennzeichnet, dass die Module (19, 20, 21, 22) Einheiten beinhalten, die programmierbar sind.

17. Gerät zur Vestibularisreizung nach einem der Ansprüche 1 bis 16, dadurch gekennzeichnet, dass die einzelnen Module (19, 20, 21, 22) durch eine gedruckte Schaltung miteinander in Wirkverbindung sind.

18. Gerät zur Vestibularisreizung nach einem der Ansprüche 1 bis 17, dadurch gekennzeichnet, dass die Elektroden (16, 17) bügelartig ausgebildet sind.

5 0 177 661 6

19. Gerät zur Vestibularisreizung nach einem der Ansprüche 1 bis 17, dadurch gekennzeichnet, dass die Elektroden (16, 17) durch ein Stirnband an den Schädel, vorzugsweise hinter den Ohren, anlegbar sind.

20. Gerät zur Vestibularisreizung nach einem der Ansprüche 1 bis 17, dadurch gekennzeichnet, dass der Elektrodenträger ein Helm ist.

21. Gerät zur Vestibularisreizung nach einem der Ansprüche 1 bis 17, dadurch gekennzeichnet, dass die Elektroden (16 und/oder 17) vorzugsweise im Schädelbereich einpflanzbar sind.

22. Gerät zur Vestibularisreizung nach einem der Ansprüche 1 bis 17, dadurch gekennzeichnet, dass die Elektroden (16, 17) an der Haut des Patienten anklebbar sind.

23. Gerät nach einem der Ansprüche 1 bis 17, dadurch gekennzeichnet, dass die Elektroden (16, 17) an der Geräteaussenseite befestigt sind und mit diesem direkt am Körper des Patienten anlegbar sind.

## Claims

1. Device for vestibular stimulation characterised in that connected in series with a current source (1) are a pulse generator (19) for generating pulses with if desired variable frequency, a pulse generator (20) to adjust the pulse length, a pulse shaper (21) to adjust the rise steepness, and a driver stage (22) to adjust the amplitude, and the driver stage (22) is connected with electrodes (16, 17) which can be applied to the skull region of a patient.

2. Device for vestibular stimulation according to claim 1 characterised in that the current source (1) is a direct current battery or an accumulator.

3. Device for vestibular stimulation according to claim 1 characterised in that the current source (1) is a mains connection.

4. Device for vestibular stimulation according to one of claims 1 to 3 characterised in that the pulse shape is trapezoidal.

5. Device for vestibular stimulation according to one of claims 1 to 3 characterised in that the pulse shape is curved.

6. Device for vestibular stimulation according to one of claims 1 to 3 characterised in that the pulse shape is triangular.

7. Device for vestibular stimulation according to one of claims 1 to 3 characterised in that the pulse shape is rectangular.

8. Device for vestibular stimulation according to one of claims 1 to 7 characterised in that the impulses are rising and falling.

9. Device for vestibular stimulation according to one of claims 1 to 7 characterised in that the impulses are of continuously even sequence.

10. Device for vestibular stimulation according to one of claims 1 to 7 characterised in that the electrodes (16, 17) emit impulses intermittently.

11. Device for vestibular stimulation according to one of claims 1 to 10 characterised in that the pulse generator (19) is variable between 1 and 10 impulses per second.

12. Device for vestibular stimulation according to one of claims 1 to 11 characterised in that the impulse generator (20) is adjustable between 100 and 300 msec.

13. Device for vestibular stimulation according to one of claims 1 to 12 characterised in that the current strength in the driver stage (22) is adjustable between 0.5 and 10 mA preferably between 0.5 and 3.8 mA.

14. Device for vestibular stimulation according to one of claims 1 to 13 characterised in that the variables are resistances (R2, R6, R11, R13) constructed as potentiometers.

15. Device for vestibular stimulation according to one of claims 1 to 14 characterised in that the device components (19, 20, 21, 22) are modules in operative relationship with one another.

16. Device for vestibular stimulation according to one of claims 1 to 15 characterised in that the modules (19, 20, 21, 22) have units which are programmable.

17. Device for vestibular stimulation according to one of claims 1 to 16 characterised in that the individual modules (19, 20, 21, 22) are in operative connection one with another by means of a printed circuit.

18. Device for vestibular stimulation according to one of claims 1 to 17 characterised in that the electrodes (16, 17) are constructed bowed.

19. Device for vestibular stimulation according to one of claims 1 to 17 characterised in that the electrodes (16, 17) can be laid by means of a headband against the skull, preferably behind the ears.

20. Device for vestibular stimulation according to one of claims 1 to 17 characterised in that the electrode carrier is a cap.

21. Device for vestibular stimulation according to one of claims 1 to 17 characterised in that the electrodes (16 and/or 17) are implantable preferably in the skull region.

22. Device for vestibular stimulation according to one of claims 1 to 17 characterised in that the electrodes (16, 17) can be adhered to the skin of the patient.

23. Device according to one of claims 1 to 17 characterised in that the electrodes (16, 17) are fixed to the outer side of the device and can be laid with this directly against the body of the patient.

## Revendications

1. Appareil pour la stimulation du vestibule, caractérisé en ce qu'au moyen d'une source de courant (1) un générateur de rythme (19) pour la formation d'impulsions, éventuellement à fréquence variable, un générateur d'impulsions (20) pour le réglage de la durée d'impulsion, un formeur d'impulsions (21) pour le réglage de la pente des flancs et un étage d'attaque (22) pour le réglage de l'amplitude sont branchés en série et l'étage d'attaque (22) est relié à des électrodes (16, 17) pouvant être fixées dans la région crânienne d'un patient.

2. Appareil pour la stimulation du vestibule suivant la revendication 1, caractérisé en ce que la source de courant (1) est une batterie à courant continu ou un accumulateur.

3. Appareil pour la stimulation du vestibule suivant la revendication 1, caractérisé en ce que la source de courant (1) est un branchement au réseau.

4. Appareil pour la stimulation du vestibule suivant l'une des revendications 1 à 3, caractérisé en ce que la forme de l'impulsion est trapézoïdale.

5. Appareil pour la stimulation du vestibule suivant l'une des revendications 1 à 3, caractérisé en ce que la forme de l'impulsion est curviligne.

6. Appareil pour la stimulation du vestibule suivant l'une des revendications 1 à 3, caractérisé en ce que la forme de l'impulsion est triangulaire.

7. Appareil pour la stimulation du vestibule suivant l'une des revendications 1 à 3, caractérisé en ce que la forme de l'impulsion est rectangulaire.

8. Appareil pour la stimulation du vestibule suivant l'une des revendications 1 à 7, caractérisé en ce que les impulsions sont en forme d'ondulations vers le haut et vers le bas.

9. Appareil pour la stimulation du vestibule suivant l'une des revendications 1 à 7, caractérisé en ce que les impulsions sont une suite de durée identique.

10. Appareil pour la stimulation du vestibule suivant l'une des revendications 1 à 7, caractérisé en ce que les électrodes (16, 17) délivrent des impulsions par à-coup.

11. Appareil pour la stimulation du vestibule suivant l'une des revendications 1 à 10, caractérisé en ce que le générateur de rythme (19) est variable entre 1 et 10 impulsions par seconde.

12. Appareil pour la stimulation du vestibule suivant l'une des revendications 1 à 11, caractérisé en ce que le générateur d'impulsions (20) est réglable entre 100 et 300 ms.

13. Appareil pour la stimulation du vestibule suivant l'une des revendications 1 à 12, caractérisé en ce que l'intensité de courant dans l'étage d'attaque (22) est réglable entre 0,5 et 10 mA, de préférence entre 0,5 et 3,8 mA.

14. Appareil pour la stimulation du vestibule suivant l'une des revendications 1 à 13, caractérisé en ce que les variables sont des résistances (R2, R6, R11, R13) sous forme de potentiomètres.

15. Appareil pour la stimulation du vestibule suivant l'une des revendications 1 à 14, caractérisé en ce que les composants (19, 20, 21, 22) de l'appareil sont des modules en liaison mutuelle interactive.

16. Appareil pour la stimulation du vestibule suivant l'une des revendications 1 à 15, caractérisé en ce que les modules (19, 20, 21, 22) renferment des unités qui sont programmables.

17. Appareil pour la stimulation du vestibule suivant l'une des revendications 1 à 16, caractérisé en ce que les modules individuels (19, 20, 21, 22) sont en liaison interactive par l'intermédiaire d'un circuit imprimé.

18. Appareil pour la stimulation du vestibule suivant l'une des revendications 1 à 17, caractérisé en ce que les électrodes (16, 17) sont en forme d'étrier.

19. Appareil pour la stimulation du vestibule suivant l'une des revendications 1 à 17, caractérisé en ce que les électrodes (16, 17) peuvent être posées sur le crâne, de préférence derrière les oreilles, au moyen d'un bandeau.

20. Appareil pour la stimulation du vestibule suivant l'une des revendications 1 à 17, caractérisé en ce que le support des électrodes est un casque.

21. Appareil pour la stimulation du vestibule suivant l'une des revendications 1 à 17, caractérisé en ce que les électrodes (16, 17) sont, de préférence, implantables dans la région crânienne.

22. Appareil pour la stimulation du vestibule suivant l'une des revendications 1 à 17, caractérisé en ce que les électrodes (16, 17) peuvent être collées sur la peau du patient.

23. Appareil pour la stimulation du vestibule suivant l'une des revendications 1 à 17, caractérisé en ce que les électrodes (16, 17) sont fixées sur l'extérieur de l'appareil et peuvent être posées avec celui-ci directement sur le corps du patient.

Fig.1